# EUROPEAN PATENT APPLICATION

(11) **EP 3 178 499 A1**
(43) Date of publication of application: **14.06.2017**
(21) Application number: 15199284.9
(22) Date of filing: 10.12.2015
(51) Int. Cl.: A61L 9/12

(54) **FRAGRANCE DEVICE AND A NOZZLE STRUCTURE OF A FRAGRANCE DEVICE**

(71) Applicant: Sevende Ky, 00140 Helsinki (FI)
(72) Inventor: Niskanen, Eero, 00140 Helsinki (FI)
(74) Representative: Berggren Oy, Helsinki & Oulu

(57) **Abstract**

The invention relates to a fragrance device (10) comprising a mixing chamber for mixing fragrance molecules and air and, an outer wall structure inside of which the mixing chamber is located, at least one opening (16) to the mixing chamber for intake air, and a nozzle structure for creating an fragrance flow. The nozzle structure for creating the fragrance flow is a honeycomb-type nozzle structure comprising several nozzle opening channels (21), through which the fragrance flow is guided from the mixing chamber of the fragrance device (10) to a desired location.

## Description

The invention relates to fragrance devices. In particular the invention relates to a fragrance device according to the preamble of claim 1 and to a nozzle structure of a fragrance device according to the preamble of claim 10.

Sense marketing is a vastly growing industry around the world, in sense marketing the idea is to use fragrances and sounds in marketing is to improve the comfort of customers and to create a more satisfying experience as well as improving the atmosphere by freshening it up. In sense marketing fragrance devices are used to spread to the atmosphere air with fragrance. The fragrance devices are thus used for sense marketing and for freshening up the atmosphere of a room. The fragrance devices are used for example in stores, in hotel lobbies, in offices etc.

In publication WO 2014148999 A1 is disclosed a device for delivering volatile substances, comprising: a vapor permeable outer enclosure, said outer enclosure being formed by a vapor permeable material layer; and a vapor impermeable inner enclosure placed inside the outer enclosure and defining an interior space for accommodating the volatile substances, said inner enclosure being formed by a second laminate comprising: a metal foil layer, and a rupturable material layer laminated onto the metal foil layer, wherein at least one die cut line is formed through the rupturable material layer. These devices are used for example as fragrance pouches containing the substance with fragrance molecules to be mixed with air and spread as an air flow to the atmosphere by the fragrance devices.

In publication EP 2826510 A1 is disclosed a volatile substance diffusing device comprising a fluid inlet being in fluid communication with a nebulizer chamber into which a nebulizing tube is extended, which is in fluid communication with a volatile substance supply tank, in which the volatile substance is released to be experienced as a mixture of fluid and volatile substance by a user approaching the scent-release device.

In publication US 2012251296 A1 is disclosed an aromatic nebulizing diffuser, comprising: a base panel; a power switch mounted on said base panel; an electric fan mounted on said base panel and electrically connected to and controllable by said power switch; a holder comprising a transverse partition board, said transverse partition board dividing said holder into an upper part and a lower part, a center opening cut through said transverse partition board, said upper part defining therein an oscillation chamber, said lower part defining a cover, and an air passage set in air communication between the space inside said cover and said oscillation chamber to provide a path for delivering air currents caused by said electric fan; a fluid container mounted in said holder, said fluid container comprising a container body, a container base fastened to a bottom side of said container body and defining with said container body a fluid storage chamber and an exhaust passage in air communication between said oscillation chamber and the atmosphere for exhaust of a mist of aromatic fluid droplets out of said oscillation chamber; an ultrasonic oscillator mounted in the opening of said holder and electrically connected to said power switch; a fluid intake control device mounted in a bottom side of said fluid container and selectively set between an open status and a close status to control delivery of a fluid from said fluid storage chamber to said oscillation chamber; and a dip tube set in said fluid container and said oscillation chamber to communicate said fluid storage chamber and said oscillation chamber.

In publication US 2005072857 A1 is disclosed a device for aromatizing a gaseous medium comprising a chamber for mixing the gaseous medium with aromatizer fumes; at least one aromatizer dispenser connected to the mixing chamber, said dispenser containing a vessel for the aromatizer and an actuating element made with a possibility to be connected to a power supply; a unit for controlling the operation of the device; and a booster for circulating the gaseous medium through the mixing chamber, which aromatizer dispenser additionally comprises a reservoir connected to the vessel for the aromatizer; a reservoir wall has at least one nozzle for injecting the aromatizer into the mixing chamber; the actuating element is embodied to produce pressure pulses in the reservoir filled with the aromatizer and arranged in the interior of the reservoir on its wall made of an insulating material.

In fragrance devices known from prior art one disadvantage has been the inaccurate spreading of the fragrance and another disadvantage has also been the difficulty of adjusting the intensity of the fragrance at the desired location.

An object of the present invention is to create a fragrance device a new type of nozzle structure.

An object of the present invention is to provide a fragrance device and a nozzle structure of a fragrance device in which disadvantages of known devices are eliminated or at least minimized.

In order to achieve the above objects and those which will come apparent later the fragrance device is characterized by the features of claim 1. The nozzle structure of the fragrance device in turn is mainly characterized by the features of claim 10. Advantageous embodiments and features are disclosed in the dependent claims.

According to the invention the fragrance device comprises a mixing chamber for mixing fragrance molecules and air and, an outer wall structure inside of which the mixing chamber is located, at least one opening to the mixing chamber for intake air, and a nozzle structure for creating an fragrance flow, wherein the nozzle structure for creating the fragrance flow is a honeycomb-type nozzle structure comprising several nozzle opening channels, through which the fragrance flow is guided from the mixing chamber of the fragrance device to a desired location.

According to an advantageous feature of the invention the nozzle structure is formed as a nozzle element forming a releasable part the outer wall structure.

According to an advantageous feature of the invention nozzle opening channels are tapered and the tapered form decreases in direction of outwards from the fragrance device i.e. in direction of the fragrance flow.

According to an advantageous feature of the invention overall open surface area of the nozzle opening channels at the outer surface of the nozzle element is at least 50 % of the overall surface area of the outer surface of the nozzle element.

According to an advantageous feature of the invention the fragrance device further comprises a grid structure located at least partially at a distance from the outer wall structure and on which grid structure a fragrance pouch with fragrance substance for providing the fragrance molecules is located

According to an advantageous feature of the invention the fragrance device further comprises a blower located at the vicinity of the inlet opening and a power source for the blower.

According to an advantageous feature of the invention the fragrance device further comprises control means connected to the blower and to the power source, which control means advantageously comprise an on-off switch, pulsing and power controls and a timer for controlling and adjusting the operation of the fragrance device and the fragrance flow generated by the fragrance device.

According to an advantageous feature of the invention control means is based on fragrance pulse principle, whereby the fragrance flow is fed in pulses.

According to an advantageous feature of the invention the nozzle element is advantageously turnably by hinges attached to the outer wall structure of the fragrance device functioning as a door.

According to the invention the nozzle structure of a fragrance device for generating a fragrance flow, which nozzle structure comprises nozzle opening channels, wherein the nozzle structure for creating the fragrance flow is a honeycomb-type nozzle structure comprising several nozzle opening channels, through which the fragrance flow is guided from the fragrance device to a desired location.

According to an advantageous feature of the invention the nozzle structure is formed as a nozzle element forming a releasable part the fragrance device.

According to an advantageous feature of the invention nozzle opening channels are tapered and the tapered form decreases in direction of outwards from the fragrance device i.e. in direction of the fragrance flow.

According to an advantageous feature of the invention form of the outlet opening of the nozzle opening channel is advantageously circular.

In the method of operating a fragrance device according to the invention a power source of the fragrance device is turned on to rotate a blower of the fragrance device, the blower provides for the intake of air through opening/openings in an inlet element of the fragrance device and that inside the fragrance device the air circulates and takes into the air flow fragrance molecules from a fragrance pouch, containing fragrance substance, located on a grid structure, and that the air flow with the fragrance molecules i.e. the fragrance flow is collected by nozzle opening channels of a nozzle element of the fragrance device and guided the desired location.

Thus the present invention relates to a fragrance device for spreading selected fragrance to a selected area in a space, or in a room.

The fragrance devices, in which fragrance pouches or packages containing the fragrance substance in liquid or gel form are used and the fragrance molecules of the fragrance substance are mixed with air and spread to the desired location as an air flow containing the fragrance molecules, which air flow is created by the fragrance device in a mixing chamber of the fragrance device, are known from prior art. In one type of fragrance devices known from prior art the fragrance pouch is located on to a grid structure located in the fragrance device, which grid structure is open upwards and downwards i.e. on both sides of the fragrance pouch such that air flow circulates on both sides of the fragrance pouch and the distribution of the fragrance molecules will be uniform, simultaneously preventing possible leaks or seeps to effect the fragrance output. The fragrance device of this type is small in size, effective and accurately adjustable and suitable for spaces of different types and sizes. The present invention relates especially to these types of fragrance devices.

The adjustment of the fragrance device is advantageously based on so called fragrance pulse principle, whereby the spreading of the fragrance is advantageously not continuous but spread in pulses, whereby the intensity of the fragrance does not exceed over the desired fragrance effect as by pulse adjustment the desired amount of fragrance molecules are guided to the desired location. This adjustment principle also spares the fragrance substance, thus one fragrance pouch lasts for a longer time compared to continuous use. Advantageously the fragrance device also comprises a timer for keeping the device on desired time.

Advantageously the fragrance device also comprises fastening means for attaching it to the desired location, for example to a shelf structure of a store. More advantageously the fastening means are magnetic such that the position of the fragrance device is also easily adjustable and thus also the direction of the fragrance flow is easily adjustable. The fastening means may also comprise a turnable assembly plate. Advantageously the fragrance device comprises quick openable means such that the fragrance pouches are easily replaceable.

According to the invention the fragrance device has a new type of nozzle structure by which more effective and more accurate fragrance output is achieved at the desired location in the space. The inventive nozzle structure increases the effectivity even 70 % compared to the type of fragrance device of the kind.

By the inventive nozzle structure the fragrance flow is throttled and its effective distance increases compared to the fragrance device of the kind.

Due to the inventive nozzle structure the air flow with the fragrance molecules i.e. the fragrance flow is guided to the desired location accurately and adjustably.

Due to the inventive nozzle structure also the intensity of the fragrance flow is adjustable such that it overcomes the possible outlet air flows from the space, if necessary, such that the fragrance flow is not guided directly off the space with the outlet air flows.

The inventive nozzle structure also guides the fragrance flow to a longer range than for example a one nozzle arrangement.

The present invention also provides for an adjustable fragrance flow in a large space into desired direction and corresponding in small space a fragrance flow with desired intensity.

According to an advantageous example the nozzle structure of the fragrance device is combined with the pulse-type flow adjustment and with direction adjustability of the fragrance device provided by the fastening means.

The nozzle structure is a honeycomb-type structured comprising several in honeycomb-form arranged advantageously tapered nozzle openings, in which the tapered form decreases in direction of outwards from the fragrance device i.e. in direction of the fragrance flow.

Advantageous dimensional features of the nozzle structure may comprise one or more of the following in any combination:
- the overall surface area of the nozzle openings is at least 50 % of the overall surface area of the nozzle element comprising the nozzle structure
- angle of the outlet edge of the nozzle opening, advantageously less than 90 °
- inlet edge form of the nozzle opening channel is rounded such that good flow properties are achieved
- form of the outlet opening of the nozzle opening channel is circular

In the following the invention is described in more detail with reference to the accompanying drawing in which

in figures 1 - 2 is schematically shown an advantageous example of a fragrance device according to the invention and

in figures 3 - 4 is schematically shown advantageous examples of the nozzle element according to the invention.

In the following description and in the accompanying drawing by same reference signs similar or corresponding parts and part-components are denoted unless otherwise mentioned.

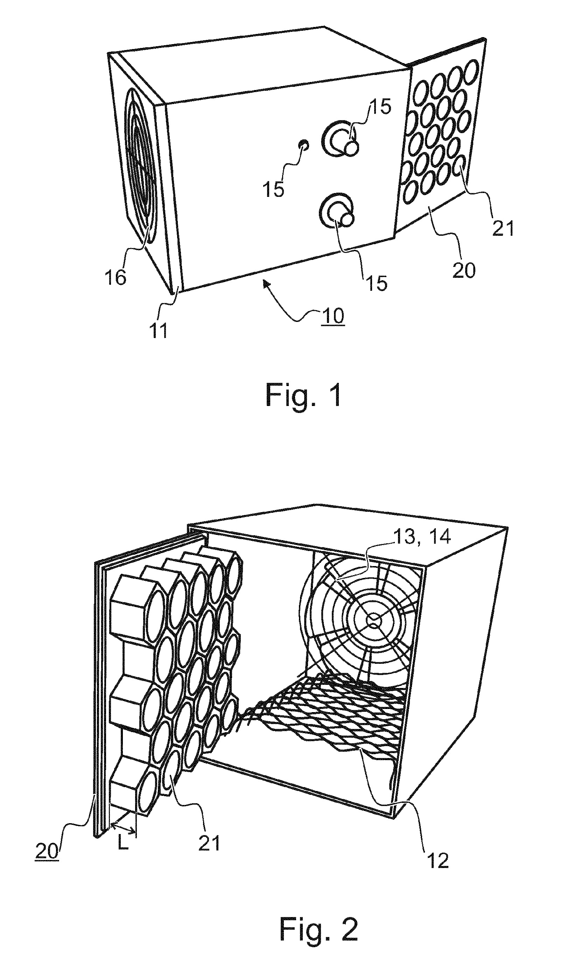

In the example of figures 1 - 2 the fragrance device 10 is a box-like construction comprising a mixing chamber inside the outer wall structure, which in this example is a box-like structure. One wall is formed as an inlet element 11 comprising inlet openings 16 for intake air. In connection with one wall control means 15 for the fragrance device 10 are located, which control means are connected to a blower 13 for intake air and to a power source 14, which is advantageously an electric motor connected to supply current source, also an accumulator or other power sources can be used. The control means 15 advantageously comprise an on-off switch, pulsing and power control and timer for controlling and adjusting the fragrance device and the fragrance flow created by the fragrance device. Advantageously at opposite wall to the inlet element 11 a nozzle element 20 forming one wall of the mixing chamber is located. The nozzle element 20 has at least one honeycomb-type nozzle structure comprising several nozzle opening channels 21 though, which the fragrance flow is guided from the fragrance device 10 to the desired location.

In the fragrance device 10 a fragrance pouch (not shown) containing the fragrance substance in liquid or gel form is located on the grid structure 12. Fragrance molecules of the fragrance substance from the fragrance pouch are mixed with air and spread to the desired location as an air flow containing the fragrance molecules i.e. as a fragrance flow created by the fragrance device 10.

The fragrance pouch is located on the grid structure 12 located on the bottom in the fragrance device 10, which grid structure 12 is open upwards and downwards i.e. on both sides of the fragrance pouch such that air flow circulates on both sides of the fragrance pouch.

The adjustment of the fragrance device 10 by the control means 15 is advantageously based on fragrance pulse principle, whereby the flow of the fragrance is fed in pulses, whereby the intensity of the fragrance does not exceed over the desired fragrance effect as by pulse adjustment the desired amount of fragrance molecules can be guided to the desired location. The control means 15 of the fragrance device 10 also may comprise a timer for keeping the fragrance device 10 on selected time.

The nozzle element 20 is advantageously turnably by hinges attached to the corresponding wall of the fragrance device 10 functioning as a door and advantageously comprising quick-opening means such that the fragrance pouches are easily replaceable. The nozzle elements 20 can also be replaceable for changing the nozzle element 20 with desired nozzle structure with for example of different diameter of the nozzle opening channels or different tapering of the nozzle opining channel 21. By the inventive nozzle structure the fragrance flow is throttled by the tapered form of the nozzle opening channels 21. The form of the outlet opening of the nozzle opening channel 21 is advantageously circular .The overall open surface area of the nozzle opening channels 21 at the outer surface of the nozzle element 20 is at least 50 % of the overall surface area of the outer surface of the nozzle element 20.

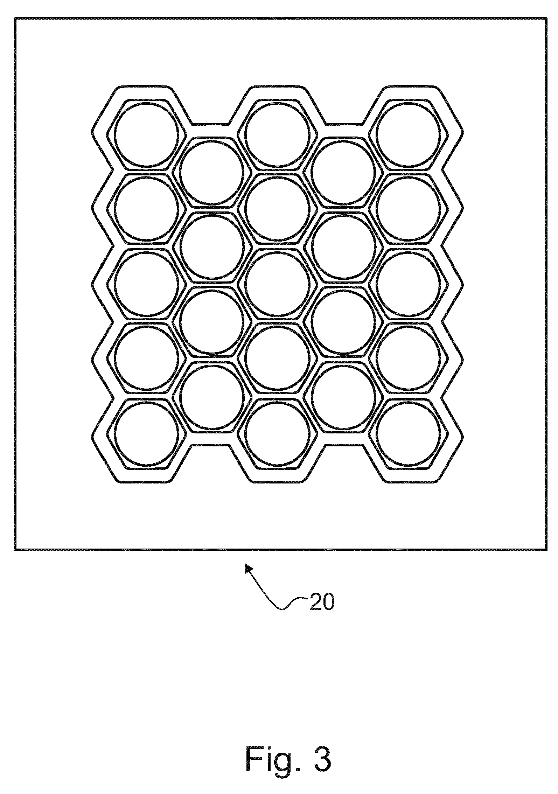

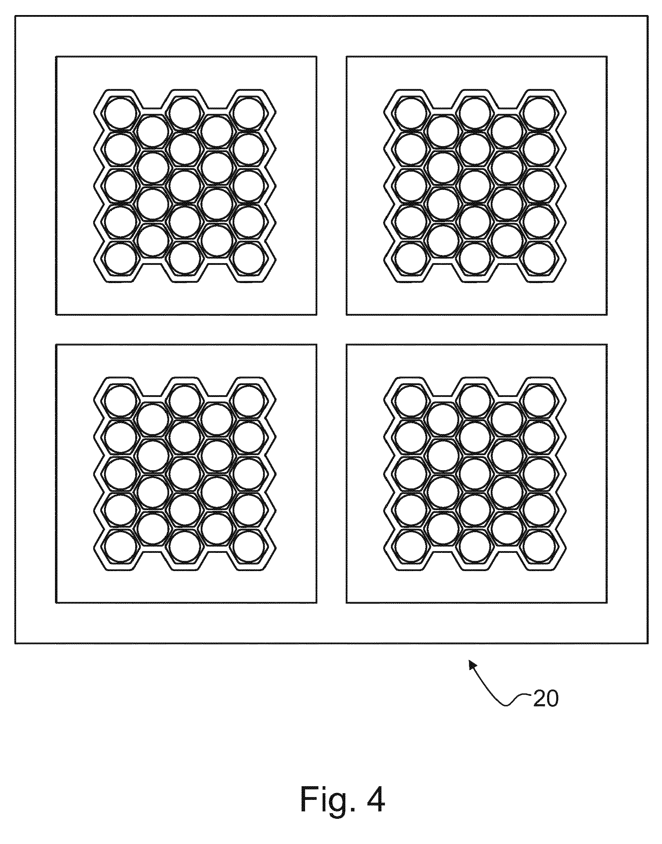

In figures 3 - 4 is shown advantageous examples of the nozzle elements 20. In figure 3 the nozzle element 20 comprises one honeycomb-type nozzle structure and in figure 4 the nozzle element 20 comprises four honeycomb-type nozzle structures. The honeycomb-type structured nozzle element 20 comprises several in honeycomb-form arranged advantageously tapered nozzle opening channels 21, in which the tapered form decreases in direction of outwards from the fragrance device i.e. in direction of the fragrance flow. The nozzle element collects the swirls of the fragrance flow and throttles the fragrance flow and directs the fragrance flow to the desired location.

When the fragrance device 10 is operated the power source 14 is turned on to rotate the blower 13 that provides for the intake of air through the opening/openings 16 in the inlet element 11. Inside the fragrance device the air circulates and takes into the air flow fragrance molecules from the fragrance pouch located on the grid structure 12, advantageously air circulates on both sides of the fragrance pouch. The air flow with the fragrance molecules i.e. the fragrance flow is collected by the nozzle opening channels 21 of the nozzle element 20 and guided by this to the desired location.

Above only some advantageous examples of the inventions has been described to which examples the invention is not to be narrowly limited and many modifications and alterations are possible within the invention.

### Reference signs used in the drawings:

- 10: fragrance device
- 11: inlet element
- 12: grid structure
- 13: blower
- 14: motor
- 15: control means
- 16: inlet opening
- 20: nozzle element
- 21: nozzle opening channel
- L: length of the nozzle opening channel

## Claims

1. Fragrance device (10) comprising a mixing chamber for mixing fragrance molecules and air and, an outer wall structure inside of which the mixing chamber is located, at least one opening (16) to the mixing chamber for intake air, and a nozzle structure for creating an fragrance flow, **characterized in that** the nozzle structure for creating the fragrance flow is a honeycomb-type nozzle structure comprising several nozzle opening channels (21), through which the fragrance flow is guided from the mixing chamber of the fragrance device (10) to a desired location.

2. Fragrance device (10) according to claim 1, **characterized in that** the nozzle structure is formed as a nozzle element (20) forming a releasable part the outer wall structure.

3. Fragrance device (10) according to claim 1 or 2, **characterized in that** nozzle opening channels (21) are tapered and the tapered form decreases in direction of outwards from the fragrance device (10) i.e. in direction of the fragrance flow.

4. Fragrance device (10) according to any of claims 1 - 3, **characterized in that** overall open surface area of the nozzle opening channels (21) at the outer surface of the nozzle element (20) is at least 50 % of the overall surface area of the outer surface of the nozzle element (20).

5. Fragrance device (10) according to any of claims 1 - 4, **characterized in that** the fragrance device (10) further comprises a grid structure (12) located at least partially at a distance from the outer wall structure and on which grid structure a fragrance pouch with fragrance substance for providing the fragrance molecules is located.

6. Fragrance device (10) according to any of claims 1 - 5, **characterized in that** the fragrance device (10) further comprises a blower (13) located at the vicinity of the inlet opening (16) and a power source (14) for the blower (13).

7. Fragrance device (10) according to any of claims 1 - 6, **characterized in that** the fragrance device (10) further comprises control means (15) connected to the blower (13) and to the power source (14), that the control means (15) advantageously comprise an on-off switch, pulsing and power controls and a timer for controlling and adjusting the operation of the fragrance device (10) and the fragrance flow generated by the fragrance device.

8. Fragrance device (10) according to claim 7, **characterized in that** control means (15) is based on fragrance pulse principle, whereby the fragrance flow is fed in pulses.

9. Fragrance device (10) according to any of claims 2 - 8, **characterized in that** the nozzle element (20) is advantageously turnably by hinges attached to the outer wall structure of the fragrance device (10) functioning as a door.

10. Nozzle structure of a fragrance device (10) for generating a fragrance flow, which nozzle structure comprises nozzle opening channels (21), **characterized in that** the nozzle structure for creating the fragrance flow is a honeycomb-type nozzle structure comprising several nozzle opening channels (21), through which the fragrance flow is guided from the fragrance device (10) to a desired location.

11. Nozzle structure according to claim 10, **characterized in that** the nozzle structure is formed as a nozzle element (20) forming a releasable part the fragrance device (10).

12. Nozzle structure according to claim 10 or 11, **characterized in that** nozzle opening channels (21) are tapered and the tapered form decreases in direction of outwards from the fragrance device (10) i.e. in direction of the fragrance flow.

13. Nozzle structure according to any of claims 10 - 12, **characterized in that** form of the outlet opening of the nozzle opening channel (21) is circular.

14. Method of operating a fragrance device according to any of claims 1 - 9, **characterized in that** in the method a power source (14) of the fragrance device (10) is turned on to rotate a blower (13) of the fragrance device, that the blower (13) provides for the intake of air through opening/openings (16) in an inlet element (11) of the fragrance device (10) and that inside the fragrance device (10) the air circulates and takes into the air flow fragrance molecules from a fragrance pouch, containing fragrance substance, located on a grid structure (12), and that the air flow with the fragrance molecules i.e. the fragrance flow is collected by nozzle opening channels (21) of a nozzle element (20) of the fragrance device (10) and guided the desired location.
